# EUROPEAN PATENT SPECIFICATION

(11) **EP 3 607 913 B1**
(45) Date of publication and mention of the grant of the patent: **07.07.2021**
(21) Application number: 18188477.6
(22) Date of filing: 10.08.2018
(51) Int. Cl.: A61F 2/36

(54) **FEMORAL CAP IMPLANT FOR HIP RESURFACING**
FEMURKAPPENIMPLANTAT FÜR HÜFTOBERFLÄCHENERSATZ
IMPLANT DE CALOTTE FÉMORALE POUR LE RESURFAÇAGE DE LA HANCHE

(43) Date of publication of application: 12.02.2020
(73) Proprietor: Mathys AG Bettlach, 2544 Bettlach (CH)
(72) Inventor: Lerf, Reto, 4513 Langendorf (CH); Liebelt, Martin, 07743 Jena (DE); Delfosse, Daniel, 3303 Jegenstorf (CH); Oberbach, Thomas, 07629 Reichenbach (DE); Witt, Carolin, 07745 Jena (DE)
(74) Representative: Gritschneder, Sebastian

(56) References cited:
- GB-A- 2 430 375
- GB-A- 2 554 844
- US-A1- 2015 335 437
- US-A1- 2016 113 771

## Description

The present invention relates to an optimized femoral cap implant for hip resurfacing with an outer, spherical articulating surface and an inner fixation surface comprising a top surface, a chamfer surface and an inner side surface. The closest prior art is document GB 2554844 A, which defines the preamble of claim 1.

At the beginning of the 21^{st} century, resurfacing with metal-on-metal bearings was a popular method of joint replacement in younger patients. However, registry data revealed higher revision rates compared to conventional hip replacement implants. The reason for the higher revision rates was considered to be caused by metallic wear debris interfering directly with the implant and also intruding into nearby regions of the human body and affecting other bodily functions.

The implications of metallic wear debris are not fully understood at the present. However, there is consensus that metallic wear of implants has to be eliminated as far as possible. A systematic analysis of failed hip resurfacing implants (Morlock M., et al. Journal of Bone Joint Surg. Am 2008, 90:89-95) showed basically four different mechanisms of failure:
The first mechanism of failure in which no acute fracture of the adjacent bone appeared was associated with excessive metal wear. The second mechanism of failure included acute fractures of the native femoral neck completely within the resurfacing head. The third mechanism of failure included acute fractures of the native femoral neck involving the rim portion of the resurfacing head. The fourth mechanism of failure involved a loosening of the acetabular cup.

It is currently assumed that at least the first three of these four mechanisms of failure are at least partially related to the design of the resurfacing head prosthesis.

Egde wear due to high cup inclination angles leading to edge loading between femoral cap head and acetabular cup was detected as one reason for the excessive wear. Edge loading can be defined as the condition where the contact area between the head and the cup is located on the rim chamfer of the acetabular cup. It was recently found that the occurrence of edge loading is a multifactorial phenomenon (O'Dwyer et al., Journal of Biomedical Materials Research Part B, Epub Sep 23, 2017). Such factors include implant surgical positioning, material of the bearing surface, implant design, surgical and patient factors such as cup and resurfacing head migration after surgery, and variations in patients' anatomy, bio-mechanics and soft tissue tension.

Fractures of the native femoral neck completely within the resurfacing head are thought to be related to osteonecrosis. Such necrosis may arise from micro-fracturing of the bone during implantation and/or may be associated with the Polymethylmethacrylate (PMMA) bone cement used for fixation of the head prosthesis.

Fractures of the native femoral neck involving the rim of the resurfacing head is attributed mainly to so called notching, i.e. cutting of the native femoral neck during reaming. This may be related to the design of the resurfacing head or surgical technique.

A prior art femoral head prosthesis is referred to in literature as the Birmingham hip resurfacing (BHR) component. This femoral head prosthesis comprises an interior cavity including a planar top portion, a frusto-conical or chamfer portion and an inner side wall. The implant is firmly cemented onto the native femoral neck. It is reported that upon hardening of excess bone cement adjacent bone tissue may be damaged leading to osteonecrosis that eventually may require revision of the implant.

It is therefore one objective of the present invention to provide an optimized design for a femoral cap to be used in hip resurfacing.

According to the present invention there is provided a femoral head resurfacing implant, or femoral cap, according to claim 1.

The femoral cap of the present invention offers the advantage to preserve as far as possible the human bone stock of the proximal femur and obtains optimized load transition from the implant to the human bone. At the same time increased wear and/or roughening of the articulation surface of the femoral cap in the case of rim loading is avoided.

In a preferred embodiment the smooth, steady transition from the outer articulation surface to the inner fixation surface of the femoral cap is established by a series of flat-radius-flat transitions.

The term "flat-radius" and/or "radius-flat transition" as used herein is to be understood as a transition between a curved portion of the surface of the femoral cap surface having a radius and a flat portion of the surface of the femoral cap. A curved portion is a surface that in a two-dimensional cross section taken in a plane parallel to and through the central axis of the femoral cap exhibits a curved line with a constant radius. A flat portion is a surface that in a two-dimensional cross section taken in a plane parallel to and through the central axis of the femoral cap exhibits a straight line without any curvature.

By designing the transition at the rim of the femoral cap i.e. between the outer articulation surface and the inner fixation surface of the femoral cap as a series of flat-radius-flat transitions a smooth transition without any grooves or ridges is obtained. With this design an optimized load distribution in particular in the rim area of the femoral cap is obtained.

In principle any number of curved and flat portions and consequently any number of radius-flat and/or flat-radius transitions may be used for designing the smooth, steady transition from the outer articulation surface to the inner fixation surface of the femoral cap. In a preferred embodiment the smooth, steady transition from the outer articulation surface to the inner fixation surface of the femoral resurfacing head includes three radius portions.

In a further preferred embodiment the smooth, steady transition from the outer articulation surface to the inner fixation surface of the femoral resurfacing head includes three radius portions and three flat portions. In this embodiment the sequential order of the transitions is as follows. The outer articulating surface is bordered by a first flat surface that is arranged oblique, preferably at an angle between 120° and 150°, with respect to the central axis of the femoral cap. This first flat portion connects to a first radial portion that in turn connects to a second flat portion that runs substantially in radial direction and orthogonal to the central rotational axis of the femoral cap. This second flat surface connects to a second radial portion that in turn connects to a third flat portion that runs substantially in axial direction and parallel to the central rotational axis of the femoral cap. This third flat surface connects to a third radial portion that in turn connects to the inner side surface of the femoral cap.

In a preferred embodiment the radius of curvature of the radius portions is equal or larger than 0.4 mm, and may preferably be equal to 0.44 mm or 0.5 mm. Transition radii that are smaller than 0.4 mm could act as stress raisers and could make the respective curved portions prone to chipping of the ceramic material. With radii that are larger than 0.4 mm the risk to create potential weak spots of the femoral cap is reduced or avoided.

Conventional femoral cap implants mostly have an inner surface that has a conical shape with the opening at the rim portion having a larger diameter than the top surface in the inner volume of the femoral cap. In particular when PMMA cement is used to fix the femoral cap, the cement has a tendency to flow out of the opening at the rim portion and may then come in contact with the surrounding bone tissue. According to a preferred embodiment of the present invention the inner side surface of the femoral cap has a non-conical surface and more preferably has a cylindrical or spherical shape. Thereby the risk of squeezing out the bone cement from the inner volume of the femoral cap is reduced.

In a preferred embodiment the length of the narrow neck portion may range between 1.0 and 3.0 mm, preferably between 1.0 and 2.0 mm, and more preferably about 1.1 mm. The narrow neck portion corresponds to a cylindrical portion in a three dimensional view of the femoral cap. With the extended neck portion stress concentration at the rim of the femoral cap during loading due to motion of the patient is reduced. Thereby also the risk of acute fracture of the native femoral neck near the rim portion of the femoral cap is minimized.

Preferably the diameter of the inner side surface may range between 25 and 55 mm, and preferably between 30.5 and 50.5 mm. The diameter of the inner side surface is larger than the diameter of the narrow neck portion by 0.2 to 0.4 mm. This design further assists in accommodating bone cement within the volume of the femoral cap and in preventing bone cement being squeezed out of the inner volume of the femoral cap.

Preferably the wall thickness of the femoral cap in the area of the inner side surface is as small as possible while at the same time offering sufficient stability and rigidity to avoid breaking of the femoral cap upon loading. Further preferably the wall thickness in the area of the inner side surface is less or equal to 3.5 mm. By designing the femoral cap with a reduced wall thickness as much human bone material of the femoral neck as possible may be preserved, which in turn reduces the risk of acute fracture of the native femoral neck.

Preferably the internal depth of the inner fixation surface of the femoral cap is as large as possible such that the contact surface between the femoral cap and the native femoral neck is increased. Further preferably the internal depth of the inner fixation surface of the femoral cap is equal or larger than 24 mm and preferably equal or larger than 26.5 mm. With this design also load concentration at the rim of the femoral cap during loading due to motion of the patient is reduced.

The chamfer portion of the inner fixation surface of the femoral cap helps in centering the femoral cap upon initial mounting of the implant by the surgeon. Preferably the chamfer angle of the chamfer portion is smaller than 45°, and preferably the chamfer angle ranges between 38° and 42° with respect to the central rotational axis of the femoral cap.

The inner fixation surface of the femoral cap may preferably comprise a central pin that extends from the top surface through the opening of the femoral cap at the rim portion. More preferably the central pin has a frusto-conical shape and has a diameter at its base that is larger than the diameter at its tip. Preferably the conical angle of the pin ranges between 1° and 3°. Further preferably the central pin of the inner fixation surface has a rounded smooth tip. The central pin is to be inserted into a channel provided in the resected femoral neck and may thereby assist in mounting and fixing the femoral cap onto the native bone of the patient.

Preferably the surface of the central pin is polished and/or non-sticky with respect to bone cement. Further preferably the length of the central pin is 10 to 20 mm larger than the depths of the top surface of the inner fixation surface. In other words the central pin extends out of the opening of the femoral cap by 10 to 20 mm. These measures help to concentrate load transition at the external cortical bone of the femoral neck. Cortical bone has a higher strength than the inner cancellous bone, and it is therefore preferred to keep the central pin short and to fix the femoral cap by the surface of the inner wall to the external cortical bone of the native femoral neck.

As mentioned above, the central pin is to be inserted into a channel provided in the native femoral neck of the patient. Preferably the diameter at the base of the central pin is slightly larger than the diameter of the channel provided to the femoral neck into which the pin is to be inserted. In this way a press-fit connection is established that enhances primary stability of the femoral cap and reduces the risk of penetration of bone cement in the bore of the natural femoral neck.

The inner fixation surface comprises the inner side surface, the chamfer surface, the top surface and the outer surface of the central pin. Preferably the transitions between these surfaces are each established by a single radius, wherein these radii are preferably as large as possible. In more detail the transition radius between the top surface and the central pin is equal or larger than 2.5 mm. The transition radius between the top surface and the chamfer surface is equal to or larger than 5.5 mm. The transition radius between the chamfer surface and the inner side surface is equal or larger than 7.5 mm. As mentioned above, small radii could act as stress raisers. With the above defined minimum radii the risk that the transition radii induce weak spots is minimized. Further, larger radii can be machined more precisely and more reproducibly and facilitate the flow of bone cement.

The inner fixation surface may further comprise internal surface structures to promote flow and distribution of PMMA bone cement and to ensure fixation of the bone cement in all directions. To this end the surface of the internal fixation surface may be roughened, such as to promote adhesion of the PMMA bone cement. Alternatively, or in addition the surface structures of the inner fixation surface may comprise channels, ribs, grooves or recesses provided to the internal fixation surface.

The inner fixation surface may further comprise internal surface structures to promote flow and distribution of PMMA bone cement. These structures may be selected from channels, grooves or recesses provided to the internal fixation surface.

The femoral cap of the present invention is preferably made from a high strength, high toughness ceramic material, and may further preferably be made from aluminum oxide, zirconium oxide, dispersion ceramics of the system aluminum oxide - zirconium oxide, or silicon nitride. By using such ceramic materials load wear is minimized and release of metal ions into the body of the patient is avoided. Thus, failures associated with excessive metal wear are substantially eliminated by manufacturing the femoral cap from ceramic material.

In order to further reduce friction and wear of the artificial hip implant, the outer, spherical articulating surface may be polished and/or may comprise a surface coating. The coating can be a relatively thin layer of material that is mounted onto the outer articulating surface of the femoral cap. The coating may be selected from one or more of a thermoplastic elastomer (TPE), a polycarbonaturethane (PCU), a polyolefin polymer, an ultra-high-molecular-weight polyethylene (UHMWPE) optionally in combination with one or more additives such as vitamin E, a polyarylketone, a polyetherketone-ketone (PEKK), or a hydrogel optionally based on PVA and/or PVD.

The invention will be further described, by way of example only, with reference to the accompanying drawings in which:
Fig. 1 shows a prior art femoral cap implant;
Fig. 2 shows an embodiment of the femoral cap implant of the present invention;
Fig. 3 shows an enlarged view of the femoral cap implant of Fig. 2.

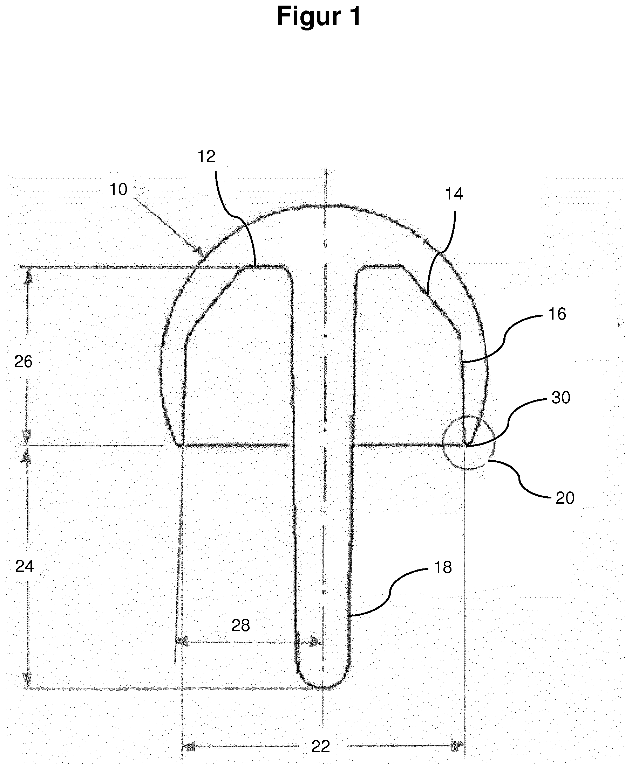

The femoral cap implant shown in Fig. 1 depicts a cross section of the so called BHR implant that is mentioned in the introductory portion of the present application. This implant represents prior art and is currently used in hip resurfacing. The prosthesis is made from a cast CoCr alloy and defines a hollow spherical cap comprising an outer spherical surface 10. The prosthesis further comprises an inner fixation surface that is mounted onto a correspondingly resected portion of the natural femoral neck of a patient. The inner fixation surface comprises a top surface 12, a chamfer surface 14 and an inner side surface 16. From the center of the top surface 12 a central pin 18 extends out of the opening 22 at the rim portion 20 of the femoral cap implant. The projecting length 24 of the central pin 18 is larger than the internal depth 26 of the inner fixation surface of the prosthesis.

The inner side surface 16 conically opens toward the opening 22 at the rim portion 20 of the prosthesis, as indicated by angle 28. The rim portion 20, i.e. the portion which comprises the transition between the outer spherical surface 10 and the inner side surface 16, is formed from a single radius portion 30 which represents a sharp transition between the inner and the outer side of the prosthesis. While this prosthesis allows for a firm fixation of the femoral cap to the resected femoral neck, it has been reported that upon hardening of excess bone cement adjacent bone tissue may be damaged leading to osteonecrosis that eventually may require revision of the implant.

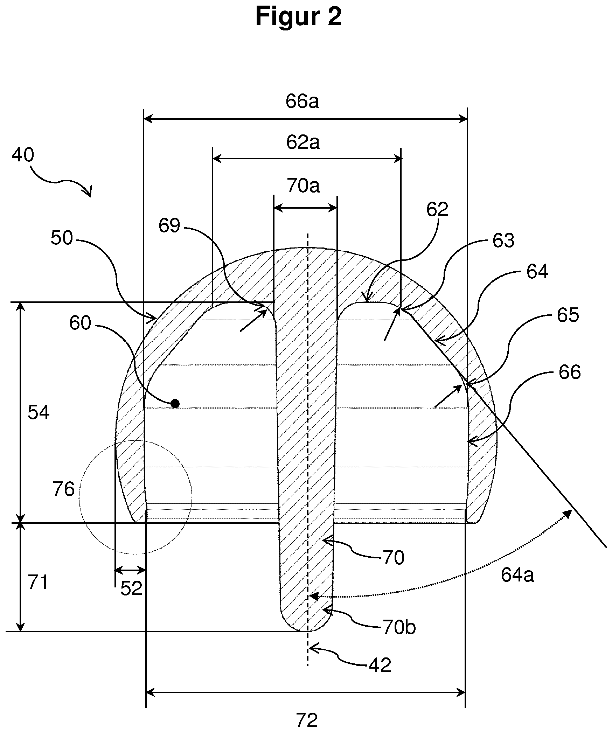

Fig. 2 is a cross sectional view of the femoral cap implant 40 of the present invention. The cross section is taken in a plane parallel to and through the central rotational axis 42 of the implant 40. The implant is made from a dispersion ceramic and defines a hollow spherical cap comprising an outer spherical surface 50 with a diameter of 48 mm. The prosthesis further comprises an inner fixation surface 60 that is to be mounted onto a correspondingly resected portion of the natural femoral neck of a patient. The inner fixation surface 60 comprises a top surface 62, a chamfer surface 64 and an inner side surface 66. From the center of the top surface 62 a central pin 70 extends out of the opening 72 of the femoral cap implant 40.

The inner top surface 62 extends perpendicular to the central rotational axis 42 of the femoral cap implant 40 and has a diameter 62a of 24 mm. The inner top surface 62 is connected to the chamfer surface 64 via radius portion 63 having transition radius of 5.5 mm. The chamfer surface is inclined towards the central rotational axis 42 by an angle 64a of 40°. The chamfer surface 64 is connected to the inner side surface 66 via radius portion 65 having transition radius of 7.5 mm. The inner side surface 66 extends parallel to the central rotational axis 42 and defines a cylindrical inner volume portion with a diameter 66a of about 40 mm. In the center of the inner volume of the femoral cap implant 40 the top surface 62 is connected to the central pin 70 via radius portion 69 having a transition radius of 2.5 mm. The central pin 70 has a slightly conical shape featuring a conical angle of 2° and a rounded tip apex 70b with a radius of about 3 mm. The diameter of the pin at its onset 70a is about 7 mm and its projecting length 71, i.e. the length by which the pin 70 projects out of the opening 72 of the femoral cap implant 40 amounts to 15 mm. The internal depth 54 of the femoral cap implant 40 amounts to 26.5 mm.

The wall thickness 52 of the femoral cap implant 40 in the area of the inner side surface varies between 2.5 and 3.5 mm and is therefore thick enough to provide sufficient stability to the implant 40 and at the same time maximizes the available space for the resected native femoral head.

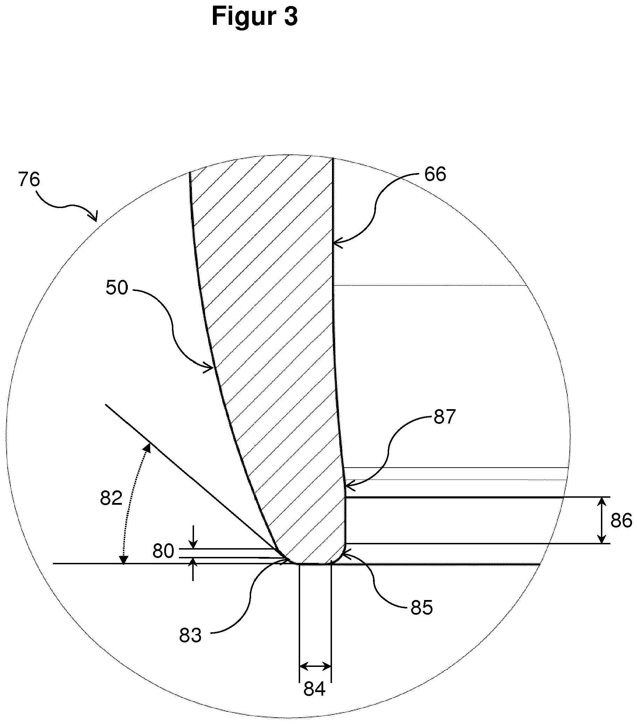

In the rim portion 76 a smooth and steady transition between the outer articulating surface 50 and the inner side surface 66 is established. An enlarged cross sectional view of the rim portion is depicted in Fig. 3.

As can be seen in Fig. 3 the smooth and steady transition between the outer articulating surface 50 and the inner side surface 66 is obtained by a series of flat-radius and radius-flat transitions, respectively. The rim portion 76 comprises three flat surfaces 80, 84, 86 and three radius portions 83, 85, 87 that are alternately arranged between the outer articulating surface 50 and the inner side surface 66. In this embodiment all three radial portions 83, 85, 87 of the rim portion 76 have an identical radius of 0.5 mm.

The outer articulating surface 50 is connected to a first flat portion 80 that is arranged at an angle 82 of 45° with respect to the second flat portion 84. The first flat portion 80 is connected to a first radial portion 83, which in turn is connected to a second flat portion 84. The second flat portion 84 is arranged perpendicular to the central rotational axis 42 of the femoral cap implant 40. The second flat portion 84 is connected to a second radial portion 85, which in turn is connected to a third flat portion 86. The third flat portion 86 is configured to run parallel to the central rotational axis 42 of the femoral cap implant 40. The third flat portion 86 is connected to a third radial portion 87, which in turn is connected to the flat inner side surface 66 of the femoral cap implant 40. The inner diameter 66a of the inner side surface 66 is 0.3 mm larger than the diameter of the third flat portion 86, respectively. Thus, the third flat portion 86 represents the narrow, cylindrical neck portion of the rim portion 76 of the femoral cap implant 40 and accordingly defines the size and diameter of the opening 72 of the rim portion 76 of the femoral cap implant 40.

## Claims

1. A femoral head resurfacing implant (40) comprising:
an outer, spherical articulating surface (50), preferably polished,
an inner fixation surface (60) comprising
a top surface (62), substantially orthogonal to a rotational center axis (42) of the femoral head resurfacing implant (40),
a chamfer surface (64) extending downwardly and outwardly from the top surface (62), and
an inner side surface (66) extending between the chamfer surface (64) and a rim portion (76) of the femoral head resurfacing implant (40),
wherein the rim portion (76) of the femoral head resurfacing implant (40) is being defined by a smooth, steady transition from the outer articulation surface (50) to the inner side surface (66) of the femoral head resurfacing implant (40),
wherein the rim portion (76) comprises a narrow neck portion (86) having cylindrical shape and having a diameter,
**characterized in that**
a diameter of the inner side surface (66) is larger than the diameter of the narrow neck portion (86) by 0.2 mm to 0.4 mm.

2. A femoral head resurfacing implant (40) in accordance with claim 1, wherein the smooth, steady transition from the outer articulation surface (50) to the inner fixation surface (66) of the femoral head resurfacing implant (40) is established by a series of flat-radius-flat transitions.

3. A femoral head resurfacing implant (40) in accordance with any of the previous claims, wherein the inner side surface (66) has a cylindrical or spherical shape.

4. A femoral head resurfacing implant (40) in accordance with any of the previous claims, wherein the wall thickness of the femoral head resurfacing implant (40) in the portion of the inner side surface (66) is less or equal to 3.5 mm.

5. A femoral head resurfacing implant (40) in accordance with any of the previous claims, wherein the depth of the inner fixation surface (60) is larger or equal to 26.5 mm.

6. A femoral head resurfacing implant (40) in accordance with any of the previous claims, wherein the chamfer angle (64a) of the chamfer portion (64) is smaller than 45°, and preferably ranges between 38° and 42° with respect to the central rotational axis (42) of the femoral head resurfacing implant (40).

7. A femoral head resurfacing implant (40) in accordance with any of the previous claims, wherein the inner fixation surface (60) has a central pin (70) that is conically formed with a conical angle of between 1° and 3° and whose diameter at the base is larger than the diameter at the tip (70b).

8. A femoral head resurfacing implant (40) in accordance with any of the previous claims, wherein the pin (70) of the inner fixation surface (60) has a rounded smooth tip (70b) and/or a surface that is non-sticky with respect to bone cement.

9. A femoral head resurfacing implant (40) in accordance with any of the previous claims, wherein the pin (70) of the inner fixation surface (60) has a length that is 10 to 20 mm larger than the internal depths of the inner fixation surface (60).

10. A femoral head resurfacing implant (40) in accordance with any of the previous claims, wherein the diameter at the base of the pin (70) is larger than the diameter of a hole in the femoral neck into which the pin is to be inserted.

11. A femoral head resurfacing implant (40) in accordance with any of the previous claims, wherein the transitions between the various sections of the inner surface (66) have radii that are as large as possible, wherein preferably the transition radius between the top surface (62) and the central pin (70) is equal or larger than 2.5 mm, and/or wherein preferably the transition radius between the chamfer portion (64) and the top surface (62) is equal to or larger than 5.5 mm, and/or wherein preferably the transition radius between the inner side surface (66) and the chamfer portion (64) is equal or larger than 7.5 mm.

12. A femoral head resurfacing implant (40) in accordance with any of the previous claims, wherein the femoral head resurfacing implant is made from high strength, high toughness ceramic material, preferably from aluminium oxide, zirconium oxide, dispersion ceramics of the system aluminium oxide - zirconium oxide, or silicon nitride.

13. A femoral head resurfacing implant (40) in accordance with any of the previous claims, wherein the outer articulating surface (50) is provided with a coating and wherein preferably the material of the coating comprises a thermoplastic elastomer, polycarbonate urethane, a polyolefin polymer, a poly aryl ketone, and/or a hydrogel.

## Patentansprüche

1. Oberschenkelkopf-Oberflächenimplantat (40), umfassend:
eine äußere, sphärische Gelenkoberfläche (50), vorzugsweise poliert,
eine innere Fixierungsoberfläche (60) umfassend
eine obere Oberfläche (62), die im Wesentlichen orthogonal zu einer Drehmittelachse (42) des Oberschenkelkopf-Oberflächenimplantats (40) ist,
eine abgeschrägte Oberfläche (64), die sich von der oberen Oberfläche (62) nach unten und nach außen erstreckt, und
eine innere Seitenoberfläche (66), die sich zwischen der abgeschrägten Oberfläche (64) und einem Randabschnitt (76) des Oberschenkelkopf-Oberflächenimplantats (40) erstreckt,
wobei der Randabschnitt (76) des Oberschenkelkopf-Oberflächenimplantats (40) durch einen glatten, stetigen Übergang von der äußeren Gelenkoberfläche (50) zu der inneren Seitenoberfläche (66) des Oberschenkelkopf-Oberflächenimplantats (40) definiert ist,
wobei der Randabschnitt (76) einen schmalen Halsabschnitt (86) mit zylindrischer Form und mit einem Durchmesser umfasst, **dadurch gekennzeichnet, dass**
ein Durchmesser der inneren Seitenoberfläche (66) um 0,2 mm bis 0,4 mm größer ist als der Durchmesser des schmalen Halsabschnitts (86).

2. Oberschenkelkopf-Oberflächenimplantat (40) nach Anspruch 1, wobei der glatte, stetige Übergang von der äußeren Gelenkoberfläche (50) zu der inneren Fixierungsoberfläche (66) des Oberschenkelkopf-Oberflächenimplantats durch eine Reihe von Flach-Radius-Flach-Übergängen hergestellt wird.

3. Oberschenkelkopf-Oberflächenimplantat (40) nach einem der vorhergehenden Ansprüche, wobei die innere Seitenoberfläche (66) eine zylindrische oder sphärische Form aufweist.

4. Oberschenkelkopf-Oberflächenimplantat (40) nach einem der vorhergehenden Ansprüche, wobei die Wandstärke des Oberschenkelkopf-Oberflächenimplantats (40) im Bereich der inneren Seitenoberfläche (66) kleiner oder gleich 3,5 mm ist.

5. Oberschenkelkopf-Oberflächenimplantat (40) nach einem der vorhergehenden Ansprüche, wobei die Tiefe der inneren Fixierungsoberfläche (60) größer oder gleich 26,5 mm ist.

6. Oberschenkelkopf-Oberflächenimplantat (40) nach einem der vorhergehenden Ansprüche, wobei der abgeschrägte Winkel (64a) des abgeschrägten Abschnitts (64) kleiner als 45° ist und vorzugsweise zwischen 38° und 42° in Bezug auf die Drehmittelachse (42) des Oberschenkelkopf-Oberflächenimplantats (40) liegt.

7. Oberschenkelkopf-Oberflächenimplantat (40) nach einem der vorhergehenden Ansprüche, wobei die innere Fixierungsoberfläche (60) einen zentralen Stift (70) aufweist, der konisch mit einem Kegelwinkel zwischen 1° und 3° geformt ist und dessen Durchmesser an der Basis größer als der Durchmesser an der Spitze (70b) ist.

8. Oberschenkelkopf-Oberflächenimplantat (40) nach einem der vorhergehenden Ansprüche, wobei der Stift (70) der inneren Fixierungsoberfläche (60) eine abgerundete glatte Spitze (70b) und/oder eine Oberfläche aufweist, die in Bezug auf Knochenzement nicht klebrig ist.

9. Oberschenkelkopf-Oberflächenimplantat (40) nach einem der vorhergehenden Ansprüche, wobei der Stift (70) der inneren Fixierungsoberfläche (60) eine Länge aufweist, die 10 bis 20 mm größer ist als die internen Tiefen der inneren Fixierungsoberfläche (60).

10. Oberschenkelkopf-Oberflächenimplantat (40) nach einem der vorhergehenden Ansprüche, wobei der Durchmesser an der Basis des Stifts (70) größer ist als der Durchmesser eines Lochs im Oberschenkelhals, in das der Stift eingesetzt werden soll.

11. Oberschenkelkopf-Oberflächenimplantat (40) nach einem der vorhergehenden Ansprüche, wobei die Übergänge zwischen den verschiedenen Abschnitten der inneren Oberfläche (66) möglichst große Radien aufweisen, wobei vorzugsweise der Übergangsradius zwischen der oberen Oberfläche (62) und dem zentralen Stift (70) gleich oder größer als 2. 5 mm ist, und/oder wobei vorzugsweise der Übergangsradius zwischen dem abgeschrägten Abschnitt (64) und der oberen Oberfläche (62) gleich oder größer als 5,5 mm ist, und/oder wobei vorzugsweise der Übergangsradius zwischen der inneren Seitenoberfläche (66) und dem abgeschrägten Abschnitt (64) gleich oder größer als 7,5 mm ist.

12. Oberschenkelkopf-Oberflächenimplantat (40) nach einem der vorhergehenden Ansprüche, wobei das Oberschenkelkopf-Oberflächenimplantat aus hochfestem, hochzähem keramischem Material, vorzugsweise aus Aluminiumoxid, Zirkoniumoxid, Dispersionskeramik des Systems Aluminiumoxid-Zirkoniumoxid oder Siliziumnitrid, gefertigt ist.

13. Oberschenkelkopf-Oberflächenimplantat (40) nach einem der vorhergehenden Ansprüche, wobei die äußere Gelenkoberfläche (50) mit einer Beschichtung versehen ist und wobei vorzugsweise das Material der Beschichtung ein thermoplastisches Elastomer, Polycarbonat-Urethan, ein Polyolefinpolymer, ein Polyarylketon und/oder ein Hydrogel umfasst.

## Revendications

1. Implant de resurfaçage de tête fémorale (40) comprenant :
une surface d'articulation sphérique externe (50), de préférence polie,
une surface de fixation interne (60) comprenant
une surface supérieure (62), sensiblement orthogonale à un axe central de rotation (42) de l'implant de resurfaçage de tête fémorale (40),
une surface chanfreinée (64) s'étendant vers le bas et vers l'extérieur à partir de la surface supérieure (62), et
une surface latérale interne (66) s'étendant entre la surface chanfreinée (64) et une partie de bord (76) de l'implant de resurfaçage de tête fémorale (40),
dans lequel la partie de bord (76) de l'implant de resurfaçage de tête fémorale (40) est définie par une transition uniforme lisse depuis la surface d'articulation externe (50) jusqu'à la surface latérale interne (66) de l'implant de resurfaçage de tête fémorale (40),
dans lequel la partie de bord (76) comprend une partie de cou étroite (86) ayant une forme cylindrique et un diamètre, **caractérisé en ce qu'**un diamètre de la surface latérale interne (66) est supérieur au diamètre de la partie de cou étroite (86) par 0,2 mm à 0,4 mm.

2. Implant de resurfaçage de tête fémorale (40) selon la revendication 1, dans lequel la transition uniforme lisse depuis la surface d'articulation externe (50) jusqu'à la surface de fixation interne (66) de l'Implant de resurfaçage de tête fémorale est établie par une série de transitions plat-rayon-plat.

3. Implant de resurfaçage de tête fémorale (40) selon l'une quelconque des revendications précédentes, dans lequel la surface latérale interne (66) a une forme cylindrique ou sphérique.

4. Implant de resurfaçage de tête fémorale (40) selon l'une quelconque des revendications précédentes, dans lequel l'épaisseur de paroi de l'implant de resurfaçage de tête fémorale (40) dans la partie de la surface latérale interne (66) est inférieure ou égale à 3,5 mm.

5. Implant de resurfaçage de tête fémorale (40) selon l'une quelconque des revendications précédentes, dans lequel la profondeur de la surface de fixation interne (60) est supérieure ou égale à 26,5 mm.

6. Implant de resurfaçage de tête fémorale (40) selon l'une quelconque des revendications précédentes, dans lequel l'angle de chanfrein (64a) de la partie chanfreinée (64) est inférieur à 45°, et de préférence compris entre 38° et 42° relativement à l'axe de rotation central (42) de l'implant de resurfaçage de tête fémorale (40).

7. Implant de resurfaçage de tête fémorale (40) selon l'une quelconque des revendications précédentes, dans lequel la surface de fixation interne (60) comporte une broche centrale (70) de forme conique d'un angle de conicité entre 1° et 3° et dont le diamètre au niveau de la base est supérieur au diamètre au niveau de la pointe (70b).

8. Implant de resurfaçage de tête fémorale (40) selon l'une quelconque des revendications précédentes, dans lequel la broche (70) de la surface de fixation interne (60) présente une pointe arrondie lisse (70b) et/ou une surface qui est non collante relativement au ciment osseux.

9. Implant de resurfaçage de tête fémorale (40) selon l'une quelconque des revendications précédentes, dans lequel la broche (70) de la surface de fixation interne (60) a une longueur 10 à 20 mm plus grand que les profondeurs internes de la surface de fixation interne (60).

10. Implant de resurfaçage de tête fémorale (40) selon l'une quelconque des revendications précédentes, dans lequel le diamètre au niveau de la base de la broche (70) est supérieur au diamètre d'un trou dans le col de fémur dans lequel la broche doit être insérée.

11. Implant de resurfaçage de tête fémorale (40) selon l'une quelconque des revendications précédentes, dans lequel les transitions entre les diverses sections de la surface interne (66) ont des rayons qui sont les plus grands possibles, de préférence le rayon de transition entre la surface supérieure (62) et la broche centrale (70) étant égal ou supérieur à 2,5 mm, et/ou de préférence le rayon de transition entre la partie chanfreinée (64) et la surface supérieure (62) étant égal ou supérieur à 5,5 mm, et/ou de préférence le rayon de transition entre la surface latérale interne (66) et la partie chanfreinée (64) étant égal ou supérieur à 7,5 mm.

12. Implant de resurfaçage de tête fémorale (40) selon l'une quelconque des revendications précédentes, dans lequel l'implant de resurfaçage de tête fémorale est réalisé en un matériau céramique de haute résistance, et de grande endurance, de préférence en oxyde d'aluminium, oxyde de zirconium, céramiques de dispersion du système oxyde d'aluminium - oxyde de zirconium, ou nitrure de silicium.

13. Implant de resurfaçage de tête fémorale (40) selon l'une quelconque des revendications précédentes, dans lequel la surface d'articulation externe (50) est dotée d'un revêtement et de préférence le matériau du revêtement comprend un élastomère thermoplastique, un uréthane de polycarbonate, un polymère de polyoléfine, une poly-aryl-cétone, et/ou un hydrogel.
